# EUROPÄISCHE PATENTSCHRIFT

(11) **EP 3 244 847 B1**
(45) Veröffentlichungstag und Bekanntmachung des Hinweises auf die Patenterteilung: **28.11.2018**
(21) Anmeldenummer: 15817808.7
(22) Anmeldetag: 18.12.2015
(51) Int. Cl.: A61F 5/11

(54) **VORRICHTUNG ZUR NAGELKORREKTUR**
DEVICE FOR NAIL CORRECTION
DISPOSITIF D'ORTHONYXIE

(30) Priorität: 12.01.2015 DE 102015100323
(43) Veröffentlichungstag der Anmeldung: 22.11.2017
(73) Patentinhaber: 3TO GmbH, 82041 Deisenhofen (DE)
(72) Erfinder: SUTOR, Norbert, 82041 Deisenhofen (DE)
(74) Vertreter: Puschmann Borchert Bardehle Patentanwälte Partnerschaft mbB
(86) Internationale Anmeldenummer: PCT/EP2015/080521
(87) Internationale Veröffentlichungsnummer: WO 2016/113073

(56) Entgegenhaltungen:
- DE-B3-102008 010 442
- DE-C2- 3 330 813
- US-A1- 2011 282 257

## Beschreibung

Die Erfindung betrifft eine Vorrichtung zur Nagelkorrektur gemäß der im Oberbegriff des Anspruches 1 angegebenen Art. Ein Verfahren zum Befestigen der Vorrichtung an einer Nagelplatte wird offenbart. Zur Behandlung von eingewachsenen oder eingerollten Nägeln, insbesondere Großzehennägeln, wurden als Alternative zur schmerzhaften und mit langen Genesungszeiten verbundenen Operation (Emmert-Plastik) verschiedene Nagelkorrekturspangen entwickelt. Diese sogenannten Orthonyxiespangen reduzieren die Krümmung der Nagelplatte und entlasten dadurch den oftmals entzündeten Nagelfalz. Die Schmerzen werden deutlich gemindert und eine Abheilung wird ermöglicht. Zum Erzielen eines nachhaltigen Therapieerfolges verbleiben die Orthonyxiespangen für einen Zeitraum von mehreren Wochen bis einigen Monaten auf der Nagelplatte des Nagels und unterstützen diesen in seinem geraden Wachstum.

Die Wirkung dieser Nagelkorrekturspangen beruht auf Rückstellkräften und Zugkräften, die außerhalb der Nagelmitte ansetzen, oder auf Kombinationen dieser beiden Mechanismen. Bei den Rückstellkräften wirken die Kräfte im Wesentlichen senkrecht zur Nagelplatte, Mit den Zugkräften, welche im Wesentlichen parallel zur Nagelplatte wirken, werden Momente an den anzuhebenden Bereichen der Nagelplatte erzeugt.

Als Nagelkorrekturspangen gibt es neben den Drahtspangen Klebespangen aus Kunststoff und Metall. Die Erfindung betrifft nun eine Weiterentwicklung einer Klebespange.

Klebespangen werden auf die Nagelplatte aufgeklebt, manchmal im vorgespannten Zustand aber auch im nicht gespannten Zustand. Im letzteren Fall wird erst nach dem Aufbringen der Klebespange auf die Nagelplatte über ein Zugelement, beispielsweise ein Draht, oder über ein Federelement eine Zugspannung erzeugt. Dabei muss sichergestellt werden, dass die Klebespange ausreichend fixiert ist, die erzeugte Zugspannung erhalten bleibt und sich nicht wieder löst. Es sind dabei Klebespangen mit Rückstellkraft und mit Zugkräften bekannt. Je nach System wird die Intensität der Spangenkraft durch die Vorauswahl verschiedener Querschnitte, Materialien oder durch Verringerung der Spangendicke, aber auch durch die mechanische Veränderung des Zugelements, beispielsweise Spannen eines Drahtes, im aufgeklebten Zustand variiert.

Aus der DE 33 30 813 C2 ist eine Vorrichtung zur Nagelkorrektur bekannt, welche zwei Haftelemente aufweist, die in Abstand zueinander auf der Nagelplatte montiert werden. Die beiden Haftelemente sind über eine Blattfeder miteinander verbunden, welche jeweils eine Rückstellkraft auf die Haftelemente in Richtung von der Nagelplatte weg erzeugt. Die Haftelemente weisen entsprechende Taschen auf, in welche die Blattfeder eingreift. Nachteilig an dieser Konstruktion ist jedoch, dass die Blattfeder entsprechend dimensioniert sein muss, um die notwendigen Rückstellkräfte zu erzeugen, und die die Blattfeder aufnehmenden Taschen ebenfalls entsprechend dimensioniert sein müssen, um die von der Blattfeder eingeleiteten Kräfte aufzunehmen und auf die Nagelplatte zu übertragen. Eine Einstellung der Rückstellkraft ist nur durch eine Auswahl der Blattfeder im Hinblick auf Material und Dimensionierung möglich. Eine stufenlose Einstellung der Rückstellkraft ist nicht möglich.

Die US 4,057,055 A offenbart ebenfalls eine Vorrichtung zur Nagelkorrektur vom Klebespangentyp. Hierbei sind zwei Haftelemente, die jeweils parallel zueinander auf einer Nagelplatte aufzubringen sind, um einen Steg zu bilden, vorgesehen. Zwischen den beiden Stegen wird ein Dehnungselement oder werden mehrere Dehnungselemente unter Spannung gespannt und dabei jeweils seitlich mit dem Steg verbunden. Für die Dehnungselemente weisen die Stege Halteknöpfe auf, in welche die Dehnungselemente eingreifen. Die Halteknöpfe halten ein Ende eines Dehnungselements in dem jeweiligen Steg. Das Dehnungselement wird durch einen Fadengummi mit festgelegten Rastpositionen gebildet. Diese Ausführungsform hat den Nachteil, dass eine stufenlos einstellbare Spannung nicht möglich ist, da der Fadengummi nur durch die vorgegebenen Rastpositionen befestigt werden kann. Zudem greifen die Dehnungselemente relativ weit beabstandet zur Oberfläche der Nagelplatte an. Dadurch trägt die Konstruktion stark auf und stört den Nutzer im täglichen Gebrauch. Des Weiteren können die Dehnungselemente verloren gehen.

Aus der EP 0 282 645 A1 ist eine Vorrichtung zur Nagelkorrektur bekannt, welche aus einer Blattfeder besteht. Die Blattfeder wird dabei vollständig auf die Nagelplatte aufgeklebt. Durch die Rückstellkraft der Blattfeder, also durch eine Kraft, welche im Wesentlichen senkrecht zur Nagelplatte wirkt, erfolgt die Korrektur. Hierbei wird vorgeschlagen, einen glasfaserverstärkten Duroplasten als Blattfeder zu verwenden. Bei dieser Konstruktion ist eine stufenlose Einstellung der Spannung nicht möglich. Durch Auswahl der Blattfeder wird jedoch die Rückstellkraft eingestellt.

Aus der DE 32 33 419 C2 ist eine Vorrichtung zur Nagelkorrektur bekannt, welche zwei Haftelemente aufweist, die zueinander beabstandet auf die Nagelplatte aufgeklebt werden. Die Haftelemente sind mit jeweils einem Haken versehen. Die Haken sind dabei gegensätzlich zueinander ausgerichtet. Durch Anbringen eines Dehnungselements an den Haken wird das Dehnungselement, nämlich ein Gummiring, gespannt. Problematisch an dieser Ausführungsform ist die sich durch die Haken ergebende Gesamthöhe der Vorrichtung, welche im täglichen Leben als störend empfunden wird. Zudem kann der Gummiring sich lösen und verloren gehen.

Aus der US 3,032,032 A und der DE 10 2007 056 614 A1 sind Vorrichtungen bekannt, welche mit der Nagelplatte formschlüssig verbunden werden.

Eine gattungsgemäße Vorrichtung zur Nagelkorrektur ist aus der US 2011/0282257 A1 bekannt, die auch den nächstliegenden Stand der Technik darstellt. Die Vorrichtung ist mit einem ersten und einem zweiten Haftelement versehen. Zwischen dem ersten und dem zweiten Haftelement ist ein elastisch wirksamer, in Wellenform gewundener Draht vorgesehen, der mit den Haftelementen an seinen Enden fest verbunden ist. Das erste und das zweite Haftelement sind dabei zueinander beabstandet angeordnet und über das Dehnungselement miteinander verbunden. Das erste und zweite Haftelement sind jeweils über eine Klebeverbindung an einem Bereich einer Nagelplatte fixierbar. Dabei bilden das erste Haftelement, das zweite Haftelement und das Dehnungselement eine unlösbare Einheit und sind einstückig ausgebildet. Das Dehnungselement ist in seiner Längsrichtung elastisch wirksam und dient als Zugelement.

Der Erfindung liegt die Aufgabe zugrunde, eine Vorrichtung zur Nagelkorrektur gemäß der im Oberbegriff des Anspruches 1 angegebenen Art derart weiterzubilden, dass unter Vermeidung der genannten Nachteile diese zum einen weiterhin flach baut, kostengünstig herzustellen ist, bei der sich Zugkräfte stufenlos einstellen lassen, und eine einfache Montage ermöglicht wird.

Diese Aufgabe wird für die Vorrichtung durch die kennzeichnenden Merkmale des Anspruches 1 in Verbindung mit seinen Oberbegriffsmerkmalen gelöst.

Die Unteransprüche bilden jeweils vorteilhafte Weiterbildungen der Erfindung.

Der Erfindung liegt die Erkenntnis zugrunde, dass sich durch eine einstückige Ausbildung sowohl eine flache Gestaltung als auch einfache, kostengünstige Herstellung ohne weiteres erreichen lässt. Zudem wird auch sichergestellt, dass ein unbeabsichtigter Verlust des Dehnungselementes und somit eine Auflösung der Zugspannung während der Behandlung auf alle Fälle nicht stattfindet. Weiterhin ergeben sich hierdurch zusätzliche konstruktive Möglichkeiten, die Vorrichtung zur Nagelkorrektur weiter zu optimieren, wie sich aus dem Folgenden ergibt.

Nach der Erfindung ist ein Halteelement zum manuellen Angreifen mit oder ohne Werkzeug für ein elastisches Dehnen des Dehnungselements während der Befestigung der Vorrichtung auf der Nagelplatte mit dem zweiten Haftelement verbunden, wobei das Halteelement mit dem ersten und zweiten Haftelement und dem Dehnungselement eine Einheit bildet und einstückig ausgebildet ist. Auf einfache Weise werden hierdurch eine Handhabe zum Spannen der Vorrichtung, also im Wesentlichen des Dehnungselements, und eine Montagehilfe bereitgestellt, welche nach Gebrauch rückstandsfrei abgeschnitten werden kann. Es bleibt zudem eine flachbauende Konstruktion als auch eine einfache Herstellung erhalten. Zudem kann das Dehnungselement einfach stufenlos gespannt werden, wodurch die Zugkraft auf die Nagelplatte individuell einstellbar ist.

Gemäß einer Ausführungsform der Erfindung ist das Halteelement über einen Verbindungsbereich mit dem zweiten Haftelement verbunden. Der Verbindungsbereich ist dabei in Form eines Steges ausgebildet. Der Querschnitt des Steges gegenüber dem Querschnitt des Halteelements ist erheblich geringer ausgeführt. Hierdurch wird erreicht, dass das Halteelement einfach von dem Haftelement abgeschnitten werden kann.

Hierbei ist vorteilhaft, wenn das Halteelement, insbesondere der Verbindungsbereich von Halteelement zu dem zweiten Haftelement, von einer Auflagefläche des zugeordneten zweiten Haftelements an einer Nagelplatte beabstandet angeordnet ist, um ein unbeabsichtigtes Verkleben des Halteelements mit der Nagelplatte zu vermeiden. Diese Maßnahme erleichtert die Montage der Vorrichtung auf eine Nagelplatte eines Nagels erheblich.

Gemäß einer vorteilhaften Weiterbildung der Erfindung ist der Querschnitt des Dehnungselements gegenüber dem jeweiligen Querschnitt des Haftelements unterschiedlich ausgebildet. Der Querschnitt kann somit individuell an die geforderten Dehnungseigenschaften angepasst werden. Beispielsweise sind das erste und zweite Haftelement gegenüber dem Dehnungselement breiter ausgebildet. Allein durch diese Maßnahme kann das Dehnungselement eine andere Dehnungseigenschaft als das erste und zweite Haftelement aufweisen. Das Dehnungselement kann auch gegenüber zumindest einem Haftelement länger ausgebildet sein. Dies hängt jedoch wesentlich von der Größe des Nagels ab.

Vorzugsweise ist das Haftelement jeweils mit einem Kleber beschichtet. Hierdurch kann das Haftelement bei Gebrauch unmittelbar auf eine Nagelplatte eines Nagels geklebt werden. Ein separates Aufbringen eines Klebers entfällt dabei.

Vorzugsweise ist die Vorrichtung mit den Haftelementen und dem Dehnungselement materialeinheitlich ausgebildet, insbesondere auch mit dem Halteelement.

Alternativ hierzu können das Dehnungselement, die beiden Haftelemente und/oder das Halteelement aus unterschiedlichen Materialien gebildet sein. Hierdurch können durch geeignete Materialwahl die Eigenschaften des jeweiligen Elements optimiert werden, Beispielsweise kann das Dehnungselement aus einem sehr elastischen Material gebildet werden, wohingegen das Haftelement aus einem weniger elastischen Material, jedoch aus einem im Hinblick auf den aufzubringenden Kleber unempfindlichen Material ausgeführt sein.

Unabhängig davon, ob die Vorrichtung materialeinheitlich oder aus unterschiedlichen Materialien gebildet ist, ist eine Ausbildung als Folienelement vorteilhaft, also mit einer im Querschnitt geringeren Höhe als Breite. Ein Folienelement ist somit flachbauend und kann einfach hergestellt werden.

Weitere vorteilhafte Eigenschaften ergeben sich dadurch, wenn das Haftelement eine größere Höhe und Breite aufweist als das Dehnungselement. Hierdurch kann das Haftelement seiner Verankerungsfunktion auf der Nagelplatte besser nachkommen, wohingegen das Dehnungselement seiner Funktion als Zugelement besser gerecht wird, wenn es im Hinblick auf seine Dehnungseigenschaften optimiert ist.

Insbesondere ist dabei das Dehnungselement beabstandet zur Auflagefläche der Haftelemente angeordnet. Hierdurch wird auf einfache Weise vermieden, dass das Dehnungselement beim Aufkleben des Haftelements in Kontakt mit dem auf dem Haftelement aufgebrachten Kleber kommt. Zudem greift das Dehnungselement beabstandet zur Nagelplatte an, wodurch das auf die Nagelplatte aufgebrachte Drehmoment erhöht wird.

Gemäß einer Ausführungsform der Erfindung sind die beiden Haftelemente jeweils formidentisch ausgebildet. Dies erleichtert die Montage, da nicht zwischen linker und rechter Seite unterschieden werden muss.

Vorzugsweise ist das Dehnungselement als Streifen ausgebildet. der sich unmittelbar entlang einer Längsachse von einem Haftelement zum anderen Haftelement erstreckt. Insbesondere ist das Dehnungselement in der Draufsicht rechteckig ausgebildet.

Ein nichterfindungsgemäßes Verfahren zum Befestigen einer Vorrichtung auf einer Finger- oder Fußnagelplatte, wie sie eben beschrieben wurde, wird offenbart. Dieses Verfahren umfasst dabei folgende Verfahrensschritte:
a. das erste Haftelement wird an der Nagelplatte an einem ersten Bereich angeklebt;
b. das Dehnungselement wird elastisch gespannt;
c. das zweite Haftelement wird unter Aufrechterhaltung der elastischen Spannung des Dehnungselements an der Nagelplatte an einem zweiten Bereich angeklebt, so dass eine dauerhafte Zugkraft an den Haftelementen anliegt;
d. das Halteelement wird abgetrennt.

Vorzugsweise wird zum Befestigen des Haftelements jeweils ein Cyanacrylat-Kleber verwendet. Ein derartiger Kleber hat sich im Zusammenwirken mit Nagelplatten bewährt.

Vorzugsweise liegt ein Satz von Vorrichtungen in jeweils unterschiedlicher Dimensionierung und/oder Dehnungseigenschaften vor. In Abhängigkeit der Größe der Nagelplatte bzw. der gewünschten Zugkraft wird eine passende Vorrichtung aus dem Satz von Vorrichtungen ausgesucht. Die ausgesuchte Vorrichtung wird dann auf der Nagelplatte wie beschrieben befestigt.

Vorzugsweise wird das Halteelement nach Befestigung der Vorrichtung auf der Nagelplatte so entfernt, dass dieses im Verbindungsbereich mit dem Haftelement abgetrennt wird. Das Halteelement kann im Hinblick auf seine Funktion des Spannens des Dehnungselements erheblich größer ausgebildet sein, da es letztendlich abgetrennt wird und die Funktion der Vorrichtung nicht beeinträchtigt. Nach der Montage wird das Halteelement einfach abgetrennt.

Das Dehnungselement kann auch im Wesentlichen quer zu einer Längsachse des Fingers bzw. der Zehe an der Nagelplatte angeordnet werden. Letzten Endes hängt die Ausrichtung der Vorrichtung bzw. des Dehnungselements von der gewünschten Zugrichtung bzw. Momenten-Aufbringung auf die Nagelplatte ab.

Die Vorrichtung kann vorzugsweise in einem Spritzverfahren und/oder Gieß-Verfahren hergestellt werden.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: eine perspektivische Ansicht auf die Vorrichtung nach der Erfindung mit zwei Haftelementen und einem die Haftelemente verbindenden Dehnungselement, wobei an einem Haftelement ein Halteelement angebracht ist;
- Fig. 2: eine Draufsicht auf die Vorrichtung von Figur 1;
- Fig. 3: eine Seitenansicht der Vorrichtung von Figur 2, und
- Fig. 4: eine Vorderansicht der Vorrichtung von Figur 3.

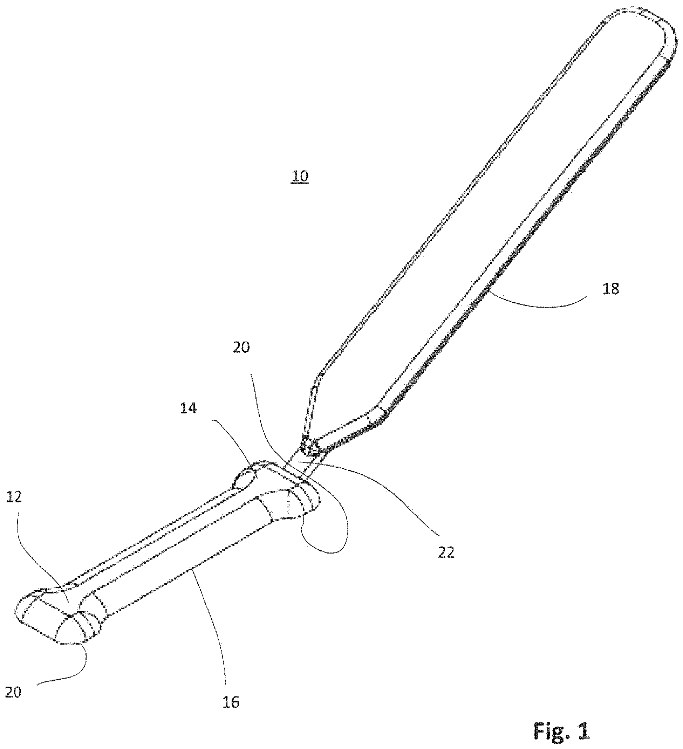

In den Fig. 1 bis 4 ist eine Vorrichtung 10 nach der Erfindung zur Nagelkorrektur eines Nagels dargestellt, Aus Gründen der Übersichtlichkeit wird jedoch die Nagelplatte nicht gezeigt, auf der die Vorrichtung 10 montiert wird.

Die Vorrichtung 10 weist ein erstes Haftelement 12, ein zum ersten Haftelement 14 beabstandetes zweites Haftelement 14, ein das erste und zweite Haftelement 12, 14 verbindendes Dehnungselement 16 und ein Halteelement 18 auf. Das erste Haftelement 12 und das zweite Haftelement 14 sind formidentisch ausgebildet und gegenüber dem Dehnungselement 16 breiter ausgeführt. Das Halteelement 18 ist über einen Steg 22 mit dem zweiten Haftelement 14 verbunden. Die Haftelemente 12 und 14 werden dabei außermittig zur Nagelplatte auf dieser befestigt.

Das erste Haftelement 12 und das zweite Haftelement 14 weisen jeweils eine Auflagefläche 20 auf, mit der sie auf der Nagelplatte im montierten Zustand aufliegen. Dabei sind die beiden Haftelemente 12, 14 mit einer Klebeschicht versehen, welche die Haftelemente 12, 14 auf der Nagelplatte fixiert. Die der Nagelplatte zugewandte Fläche der Klebeschicht bildet dabei jeweils die Auflagefläche 20 des ersten und zweiten Haftelements 12, 14.

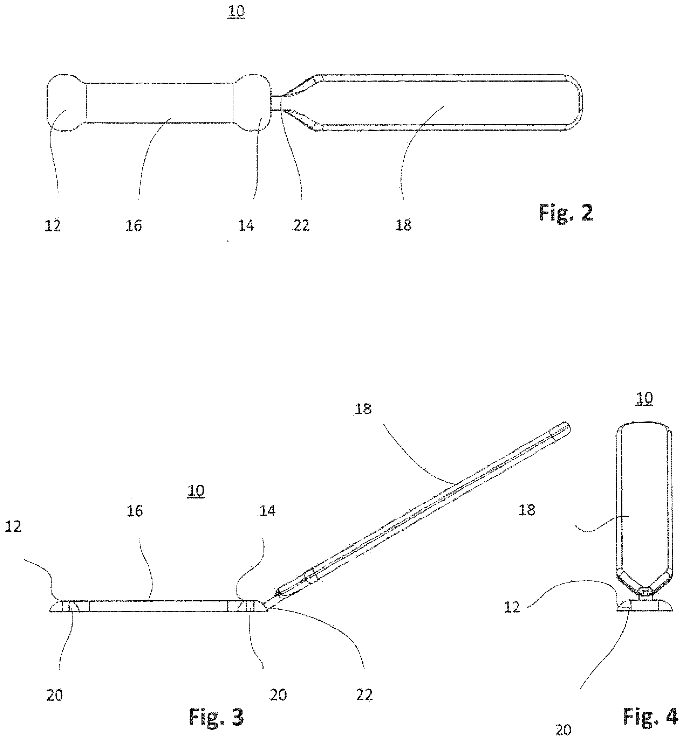

Das erste Haftelement 12 und das zweite Haftelement 14 bilden zusammen mit dem Dehnungselement 16 eine unlösbare Baueinheit. Diese unlösbare Baueinheit hat eine in der Draufsicht knochenförmige Grundform, siehe Fig. 2. Das Dehnungselement 16 ist in der Draufsicht rechteckig ausgebildet. Mit anderen Worten: das Dehnungselement 16 ist als Streifen ausgebildet, der sich unmittelbar entlang einer Längsachse von einem Haftelement 12 zum anderen Haftelement 14 erstreckt. Jeweils seitlich an das Dehnungselement schließen sich die einander formidentisch ausgebildeten Haftelemente 12, 14 an. Die Haftelemente 12, 14, aber auch das Dehnungselement 16 sind materialeinheitlich einstückig ausgebildet. Die Vorrichtung 10 kann dabei aus thermoplastischem Polyurethan, PVC oder Gummi bestehen. Durch die Formgebung des Dehnungselements 16, welches schmäler ist und eine geringere Höhe aufweist als die Haftelemente 12, 14 ist das Dehnungselement 16 gegenüber den beiden Haftelementen 12, 14 elastischer. Zudem ist das Dehnungselement 16 von der Auflagefläche 20 gering beabstandet, was in der Zeichnung nicht sichtbar ist, da die Auflösung zu gering ist.

Alternativ hierzu kann das Dehnungselement 16 auch aus einem elastischeren Material als die Haftelemente 12, 14 ausgebildet sein. Derartige unterschiedliche Materialien in einer unlösbaren Baueinheit können in einem kombinierten Spritz-Gieß-Verfahren ohne weiteres umgesetzt werden. Hierdurch kann ebenfalls die folienartige Vorrichtung 10 entstehen.

Die Haftelemente 12, 14 und das Dehnungselement 16 sind in Richtung Nagelplatte seitlich jeweils verrundet und bilden zur Auflagefläche 20 eine umlaufende Kante. Das Dehnungselement 16 schließt oben bündig an die Haftelemente 12, 14 an. Alternativ kann das Dehnungselement 16 auch leicht nach unten bezogen auf die obere Fläche des Haftelements 12, 14 versetzt sein. Zudem ist das Dehnungselement 16 leicht beabstandet zu der Auflagefläche 20 der seitlich vorgesehenen Haftelemente 12, 14 angeordnet. Hierdurch wird auf einfache Weise ein Verkleben des Dehnungselementes 16 mit der Nagelplatte vermieden, beispielsweise wenn Kleber durch den bei der Montage auf die Haftelemente 12, 14 aufgebrachten Druck seitlich austritt.

An das Haftelement 14 schließt sich der Steg 22 als Verbindungsbereich für ein Halteelement 18 an. Das Halteelement 18 ist ebenfalls folienartig ausgebildet. läuft spitz auf den Steg 22 zu und ist zusammen mit dem Steg 22 kräftig genug ausgeführt, um das Dehnungselement 16 über das Haftelement 14 und den Steg 22 bei der Montage der Vorrichtung 10 zu spannen, wie weiter unten noch näher ausgeführt wird. Dabei sind das Halteelement 18 und der Steg 22 mit dem zweiten Haftelement 14 materialeinheitlich ausgebildet. Je nach Anwendungsfall kann das erste Haftelement 12, das Dehnungselement 16, das zweite Haftelement 14, der Steg 22 und das Halteelement 18 aus dem gleichen Material oder verschiedenen Materialien hergestellt sein. Entscheidend ist die Möglichkeit, eine Zugspannung im Dehnungselement 16 zu erzeugen, welche durch Spannen des Dehnungselements 16 über das Halteelement 18, den Steg 22 und über das zweite Haftelement 14 initiiert wird.

Der Steg 22 ist ebenfalls von der Auflagefläche 20 des Haftelements 14 beabstandet angeordnet. Auch dadurch wird vermieden, dass der Steg 22, aber auch das Halteelement 18, aus Versehen durch seitlich angeordneten oder bei der Montage seitlich austretenden Kleber mit der Nagelplatte verbunden wird.

Bei der Montage der Vorrichtung 10 auf eine Nagelplatte des Nagels wird dabei wie folgt vorgegangen.

Zur Behandlung des eingewachsenen Nagels einer Zehe sollen die Nagelränder minimal angehoben werden, um den Nagelfalz zu entlasten. Dabei liegt ein Satz von Vorrichtungen 10 in jeweils unterschiedlichen Dimensionierungen und/oder mit unterschiedlichen Dehnungseigenschaften vor. In Abhängigkeit der Größe der Nagelplatte und der gewünschten Zugkraft wird eine passende Vorrichtung 10 aus dem Satz von Vorrichtungen 10 ausgewählt.

Anschließend wird zunächst das Haftelement 12 mit einem schnell aushärtenden Klebstoff, wie beispielsweise Cyanacrylat-Klebstoff, versehen. Nach Fixierung des Haftelements 12 wird auch das Haftelement 14 mit aushärtbarem Kleber versehen und in entsprechend der weiter unten skizzierten Weise auf der Nagelplatte befestigt. Alternativ hierzu kann der Kleber auch bereits auf den Haftelementen 12, 14 angeordnet und durch eine Schutzfolie abgedeckt sein. In diesem Fall muss zunächst die Schutzfolie entfernt werden, bevor die Haftelemente 12, 14 auf die Nagelplatte aufgebracht werden.

Dabei wird zunächst das Haftelement 12 außerhalb der Mitte der Nagelplatte fixiert, indem dieses so lange an die Nagelplatte angedrückt wird, bis der Kleber aushärtet. Anschließend wird das Halteelement 18 ergriffen und an dem Halteelement 18 angezogen. Das Dehnungselement 16 wird dabei stufenlos gespannt und eine gewünschte Zugkraft erzeugt. Die Zugkraft greift außermittig an der Nagelplatte zunächst über das Haftelement 12 an. Im gespannten Zustand des Dehnungselements 16 wird das zweite Haftelement 14 auf die Nagelplatte aufgebracht und dort fixiert, indem das zweite Haftelement 14 solange gegen die Nagelplatte angedrückt wird, bis der Kleber ausgehärtet ist. Hierbei wird vermieden, dass sich die vorher erzeugte Spannung des Dehnungselements 16 löst. Anschließend wird das Halteelement 18 von dem zweiten Haftelement 14 getrennt. Hierfür wird nahe am zweiten Haftelement 14 der Steg 22 mit einer Schnitteinrichtung, z. B. einer Schere, einem Seitenschneider oder einem Messer, durchtrennt.

Nach Aufbringen der Vorrichtung 10 wirkt eine dauerhafte Zugspannung zwischen den beiden Haftelementen 12, 14 durch das elastisch gespannte Dehnungselement 16. Dadurch wirkt auf den Nagel seitlich jeweils ein Biegemoment, da beide Haftelemente 12, 14 über das Dehnungselement 16 unter Zugspannung gesetzt sind. Beide Biegemomente werden somit durch das gespannte Dehnungselement 16 erzeugt. Durch die Biegemomente auf die Nagelplatte werden die Nagelränder angehoben und der Nagelfalz entlastet. Durch das leichte Anheben der Nagelplatte verlässt die Nagelplatte mit der Zeit sukzessive den eingewachsenen Zustand.

Durch ihre schnelle und einfache Anwendung wird diese Vorrichtung 10 zur Nagelkorrektur der Anforderung nach Wirtschaftlichkeit in der ärztlichen, podologischen und kosmetischen Praxis besonders gerecht.

Durch die Vermeidung von Drahthäkchen, welche beim Einhängen in den oftmals entzündeten Nagelfalz stets ein Verletzungsrisiko darstellen, ist die Anwendung dieser Vorrichtung 10 wesentlich sicherer als herkömmliche Drahtspangen. Die erfindungsgemäße Vorrichtung 10 zur Nagelkorrektur kann daher auch anhand einer mitgelieferten Gebrauchsanweisung angewendet werden.

Die Zugkraft der Vorrichtung 10, und damit das Biegemoment auf den Nagel, kann stufenlos und in Absprache mit dem Patienten eingestellt werden.

Die Abrundung der Oberfläche der Vorrichtung 10 ermöglicht dem Therapeuten, die erfindungsgemäße Vorrichtung 10 zur Nagelkorrektur direkt am Rand der Nagelplatte anzubringen. Die therapeutische Wirkung der Vorrichtung 10 wird durch die Angriffspunkte möglichst weit außerhalb der Nagelmitte optimiert.

Durch die erfindungsgemäße Ausbildung wird eine einfache, kostengünstig herzustellende und stufenlos einstellbare Vorrichtung 10 zur Nagelkorrektur bereitgestellt. Durch die folienartige Gestaltung und die abgerundeten Flächen wird eine Behinderung während der Behandlung im täglichen Gebrauch vermieden. Besonders vorteilhaft ist dabei die geringe vertikale Erstreckung der Vorrichtung 10. Durch die einteilige unlösbare Baueinheit wird vermieden, dass Einzelteile verloren gehen. Durch die beiden Haftelemente 12 und 14 ist ein einfaches Handling, insbesondere ein Spannen ohne Werkzeug über das Halteelement, möglich. Der Ort der Befestigung kann individuell eingestellt werden, je nachdem wo das Biegemoment in die Nagelplatte eingeleitet werden soll. Die neue erfindungsgemäße Vorrichtung 10 zur Nagelkorrektur bildet somit eine optimale Ergänzung und Weiterentwicklung der bisher bekannten Klebespangen.

### Bezugszeichenliste

- 10: Vorrichtung
- 12: Haftelement - links
- 14: Haftelement - rechts
- 16: Dehnungselement
- 18: Halteelement
- 20: Auflagefläche
- 22: Steg, Verbindungsbereich

## Patentansprüche

1. Vorrichtung (10) zur Nagelkorrektur mit einem ersten Haftelement (12) und einem zweiten Haftelement (14), mit einem zwischen dem ersten und dem zweiten Haftelement (12, 14) elastisch wirksamen Dehnungselement (16), wobei das erste Haftelement (12) und das zweite Haftelement (14) zueinander beabstandet angeordnet und über das Dehnungselement (16) miteinander verbunden sind, und wobei das erste und zweite Haftelement (12, 14) jeweils über eine Klebeverbindung an einem Bereich einer Nagelplatte fixierbar sind, wobei das erste Haftelement (12), das zweite Haftelement (14) und das Dehnungselement (16) eine unlösbare Einheit bilden und einstückig ausgebildet sind, und wobei das Dehnungselement (16) in seiner Längsrichtung elastisch wirksam ist und als Zugelement dient, **dadurch gekennzeichnet, dass** ein Halteelement (18) zum manuellen Angreifen mit oder ohne Werkzeug für ein elastisches Dehnen des Dehnungselements (16) während der Befestigung der Vorrichtung (10) auf der Nagelplatte mit dem zweiten Haftelement (14) verbunden ist, wobei das Halteelement (18) mit dem erstem und zweiten Haftelement (12, 14) und dem Dehnungselement (16) eine Einheit bildet und einstückig ausgebildet ist.

2. Vorrichtung nach Anspruch 1, **dadurch gekennzeichnet, dass** das Halteelement (18) über einen Verbindungsbereich (22) mit dem zweiten Haftelement (14) verbunden ist, der Verbindungsbereich in Form eines Steges (22) ausgebildet ist, und der Querschnitt des Steges (22) gegenüber dem Querschnitt des Halteelementes (18) erheblich geringer ausgebildet ist.

3. Vorrichtung nach einem der Ansprüche 1 oder 2, **dadurch gekennzeichnet, dass** das Halteelement (18), insbesondere der Verbindungsbereich (22) von Halteelement (18) zu dem zweiten Haftelement (14), von einer Auflagefläche (20) des zugeordneten zweiten Haftelements (14) an einer Nagelplatte beabstandet angeordnet ist, um ein unbeabsichtigtes Verkleben des Halteelements (18) mit der Nagelplatte zu vermeiden.

4. Vorrichtung nach einem der vorangehenden Ansprüche, **dadurch gekennzeichnet, dass** der Querschnitt des Dehnungselements (16) gegenüber dem jeweiligen Querschnitt des Haftelements (12, 14) unterschiedlich ausgebildet ist.

5. Vorrichtung nach Anspruch 4, **dadurch gekennzeichnet, dass** das erste und zweite Haftelement (12, 14) gegenüber dem Dehnungselement (16) breiter ausgebildet sind

6. Vorrichtung nach einem der vorangehenden Ansprüche, **dadurch gekennzeichnet, dass** das Haftelement (12, 14) mit einem Kleber beschichtet ist.

7. Vorrichtung nach einem der vorangehenden Ansprüche, **gekennzeichnet durch** eine materialeinheitliche Ausbildung der Haftelemente (12, 14) und des Dehnungselements (16), insbesondere auch des Halteelements (18).

8. Vorrichtung nach einem der vorangehenden Ansprüche 1 bis 6, **dadurch gekennzeichnet, dass** das Dehnungselement (16), die beiden Haftelemente (12, 14) und/oder das Halteelement (18) aus unterschiedlichen Materialien gebildet sind.

9. Vorrichtung nach einem der vorangehenden Ansprüche, **dadurch gekennzeichnet, dass** zumindest das Dehnungselement (16) aus thermoplastischem Polyurethan, PVC oder Gummi besteht.

10. Vorrichtung nach einem der vorangehenden Ansprüche, **gekennzeichnet durch** eine Ausbildung als Folienelement, also mit einer Ausbildung, die im Querschnitt eine geringere Höhe als Breite aufweist.

11. Vorrichtung nach einem der vorangehenden Ansprüche, **dadurch gekennzeichnet, dass** das Haftelement (12, 14) eine größere Höhe, Dicke und Breite aufweist als das Dehnungselement (16).

12. Vorrichtung nach einem der vorangehenden Ansprüche, **dadurch gekennzeichnet, dass** das Dehnungselement (16) beabstandet zur Auflagefläche (20) des Haftelements (12, 14) angeordnet ist.

13. Vorrichtung nach einem der vorangehenden Ansprüche, **dadurch gekennzeichnet, dass** das Dehnungselement (16) als Streifen ausgebildet ist, der sich unmittelbar entlang einer Längsachse von einem Haftelement (12, 14) zum anderen Haftelement (12, 14) erstreckt, insbesondere das Dehnungselement in der Draufsicht rechteckig ausgebildet ist.

## Claims

1. Device (10) for nail correction having a first adhesive element (12) and a second adhesive element (14), with an expansion element (16) which provides an elastic effect between said first and second adhesive elements (12, 14), which first adhesive element (12) and second adhesive element (14) are arranged at a distance from each other and are connected to each other via said expansion element (16), and which first and second adhesive elements (12, 14) can each be bonded to an area of a nail plate via an adhesive connection, with said first adhesive element (12), said second adhesive element (14) and said expansion element (16) forming an inseparable unit and being designed as a single piece, and with said expansion element (16) acting elastically in its longitudinal direction and serving as a tension element, **characterized in that** a holding element (18) is connected to the second adhesive element (14), for manual engagement with or without the use of a tool, and is adapted to elastically expand said expansion element (18) as the device (10) is being attached to the nail plate, with said holding element (18) forming a unit with said first and second adhesive elements (12, 14) and said expansion element (16) and being formed as a single piece.

2. Device according to claim 1, **characterized in that** said holding element (18) is connected to said second adhesive element (14) via a connecting area (22), which connecting area is designed as a web (22), and the cross-section of said web (22) is considerably smaller than the cross-section of said adhesive element (18).

3. Device according to one of claims 1 or 2, **characterized in that** said holding element (18), in particular the connecting area (22) from said holding element (18) to said second adhesive element (14), is arranged on a nail plate at a distance from a contact surface (20) of the associated second adhesive element (14) so as to prevent any accidental bonding of said holding element (18) to the nail plate.

4. Device according to any one of the preceding claims, **characterized in that** the cross-section of said expansion element (15) is different from the cross-section of the respective adhesive element (12, 14).

5. Device according to claim 4, **characterized in that** said first and second adhesive elements (12, 14) are wider than said expansion element (16),

6. Device according to any one of the preceding claims, **characterized in that** said adhesive element (12, 14) is coated with an adhesive.

7. Device according to any one of the preceding claims, **characterized in that** said adhesive elements (12, 14) and said expansion element (16), in particular also said holding element (18), are all made from the same material.

8. Device according to any one of the preceding claims, **characterized in that** said expansion element (16), said two adhesive elements (12, 14) and/or said holding element (18) are made from different materials.

9. Device according to any one of the preceding claims, **characterized in that** at least said expansion element (16) is made from thermoplastic polyurethane, PVC or rubber.

10. Device according to any one of the preceding claims, **characterized in that** it is formed as a film element, i.e. it is of a design which has a lower height than width in cross section.

11. Device according to any one of the preceding claims, **characterized in that** said adhesive element (12, 14) is of greater height, thickness and width than said expansion element (16),

12. Device according to any one of the preceding claims, **characterized in that** said expansion element (16) is arranged at a distance from the contact surface (20) of the adhesive element (12, 14).

13. Device according to any one of the preceding claims, **characterized in that** the expansion element (16) is formed as a strip which extends directly along a longitudinal axis from the one adhesive element (12, 14) to the respective other adhesive element (12, 14), and that in particular said expansion element is rectangular in plan view.

## Revendications

1. Dispositif (10) de correction d'ongle comprenant un premier élément adhésif (12) et un deuxième élément adhésif (14), un élément extensible (16) agissant de manière élastique entre le premier et le deuxième élément adhésif (12, 14), dans lequel le premier élément adhésif (12) et le deuxième élément adhésif (14) sont agencés à distance l'un de l'autre et sont reliés l'un à l'autre par l'intermédiaire de l'élément extensible (16), et dans lequel le premier et le deuxième élément adhésif (12, 14) peuvent être fixés à une zone d'une plaque d'ongle respectivement par l'intermédiaire d'une liaison adhésive, dans lequel le premier élément adhésif (12), le deuxième élément adhésif (14) et l'élément extensible (16) forment une unité inséparable et sont réalisés d'une seule pièce, et dans lequel l'élément extensible (16) agit élastiquement dans sa direction longitudinale et sert d'élément de traction, **caractérisé en ce qu'**un élément de retenue (18) à saisir manuellement avec ou sans outil pour une extension élastique de l'élément extensible (16) pendant la fixation du dispositif (10) sur la plaque d'ongle est relié au deuxième élément adhésif (14), dans lequel l'élément de retenue (18) forme une unité avec le premier et le deuxième élément adhésif (12, 14) et l'élément extensible (16) et est réalisé d'une seule pièce.

2. Dispositif selon la revendication 1, **caractérisé en ce que** l'élément de retenue (18) est relié au deuxième élément adhésif (14) par l'intermédiaire d'une zone de liaison (22), la zone de liaison est réalisée sous la forme d'un élément jointif (22), et la section transversale de l'élément jointif (22) est considérablement plus petite que la section transversale de l'élément de retenue (18).

3. Dispositif selon l'une quelconque des revendications 1 ou 2, **caractérisé en ce que** l'élément de retenue (18), en particulier la zone de liaison (22) de l'élément de retenue (18) au deuxième élément adhésif (14), est agencé à distance d'une surface d'appui (20) du deuxième élément adhésif (14) associé sur une plaque d'ongle, afin d'éviter un collage involontaire de l'élément de retenue (18) à la plaque d'ongle.

4. Dispositif selon l'une quelconque des revendications précédentes, **caractérisé en ce que** la section transversale de l'élément extensible (16) est différente de la section transversale respective de l'élément adhésif (12, 14).

5. Dispositif selon la revendication 4, **caractérisé en ce que** le premier et le deuxième élément adhésif (12, 14) sont plus larges que l'élément extensible (16).

6. Dispositif selon l'une quelconque des revendications précédentes, **caractérisé en ce que** l'élément adhésif (12, 14) est revêtu d'une colle.

7. Dispositif selon l'une quelconque des revendications précédentes, **caractérisé en ce que** les éléments adhésifs (12, 14) et l'élément extensible (16), en particulier également l'élément de retenue (18), sont réalisés d'un seul tenant.

8. Dispositif selon l'une quelconque des revendications précédentes 1 à 6, **caractérisé en ce que** l'élément extensible (16), les deux éléments adhésifs (12, 14) et/ou l'élément de retenue (18) sont formés de différents matériaux.

9. Dispositif selon l'une quelconque des revendications précédentes, **caractérisé en ce qu'**au moins l'élément extensible (16) est constitué de polyuréthane thermoplastique, de PVC ou de caoutchouc.

10. Dispositif selon l'une quelconque des revendications précédentes, **caractérisé en ce qu'**il est réalisé sous la forme d'un élément en feuille, par conséquent avec une configuration qui présente en section transversale une hauteur inférieure à la largeur.

11. Dispositif selon l'une quelconque des revendications précédentes, **caractérisé en ce que** l'élément adhésif (12, 14) présente une hauteur, une épaisseur et une largeur supérieure à celles de l'élément extensible (16).

12. Dispositif selon l'une quelconque des revendications précédentes, **caractérisé en ce que** l'élément extensible (16) est agencé à distance de la surface d'appui (20) de l'élément adhésif (12, 14).

13. Dispositif selon l'une quelconque des revendications précédentes, **caractérisé en ce que** l'élément extensible (16) est réalisé sous la forme d'une bande qui s'étend directement le long d'un axe longitudinal d'un élément adhésif (12, 14) à l'autre élément adhésif (12, 14), en particulier l'élément extensible est rectangulaire en vue en élévation.
